(19) **Europäisches Patentamt / European Patent Office / Office européen des brevets**

(11) **EP 4 238 547 A1**

(12) # EUROPEAN PATENT APPLICATION
published in accordance with Art. 153(4) EPC

(43) Date of publication:
**06.09.2023 Bulletin 2023/36**

(21) Application number: **21886243.1**

(22) Date of filing: **26.10.2021**

(51) International Patent Classification (IPC):
***A61K 8/362*** (2006.01)   ***A61K 8/46*** (2006.01)

(52) Cooperative Patent Classification (CPC):
**A61K 8/362; A61K 8/46; A61Q 5/04**

(86) International application number:
**PCT/JP2021/039567**

(87) International publication number:
**WO 2022/092125 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.10.2020 PCT/JP2020/040729**

(71) Applicants:
• **Sasaki Chemical Co., Ltd.**
**Tokyo, 170-0005 (JP)**

• **Oriental Chemical Co., Ltd.**
**Itami-shi, Hyogo, 664-0837 (JP)**

(72) Inventors:
• **TSUJINO, Yoshio**
**Kobe-shi, Hyogo 658-0003 (JP)**
• **TANAKA, Yoshinobu**
**Kobe-shi, Hyogo 658-0021 (JP)**

(74) Representative: **Bajjon, Alexander et al**
**Schlich**
**9 St. Catherine's Road**
**Littlehampton, West Sussex BN17 5HS (GB)**

(54) **HAIR COSMETIC AND METHOD FOR PRODUCING SAME, AND HAIR QUALITY IMPROVING METHOD AND HAIR STYLE FORMING METHOD USING SAID HAIR COSMETIC**

(57)   A hair cosmetic according to the present invention contains, as an active ingredient, a composite of an amino thiol and an organic acid. The amino thiol is at least one compound selected from the group consisting of cysteamine and a salt thereof, and the organic acid is at least one compound selected from the group consisting of fumaric acid, fumarate, maleic acid, and maleate. Such amino thiol and organic acid have been conventionally used as cosmetic materials, and ensure improved safety for users.

EP 4 238 547 A1

**Description**

Technical Field

The present invention relates to a hair cosmetic and a method for producing the same, as well as a method for improving hair quality and a method for forming a hairstyle using the hair cosmetic.

Background Art

Conventionally, various methods and means for setting hairstyles have been proposed. For example, a permanent waving agent (hereinafter may be referred to as a "perming agent") including a first agent mainly composed of a reducing agent such as thioglycolate, cysteine, cysteamine, and sodium sulfite, and a second agent mainly composed of an oxidizing agent such as sodium bromate and hydrogen peroxide is used for forming waves in hair or straightening frizzled hair. The perming agent is an agent with which, after winding hair around a rod, cystine bonds in keratin fibers constituting the hair are broken by the first agent, and in this state, the second agent is used to reform the cystine bonds into a desired shape, thus forming permanent waves (hereinafter may simply be referred to as "perm").

However, due to, for example, chemical damage caused by a perming agent or a hair coloring agent, physical damage caused by brushing or a hair dryer, and damage resulting from hair being exposed to sunlight for an extended period of time, keratin fibers gradually soften, thus making it difficult to perm hair using the conventional perming agent. That is, damaged hair and healthy hair may be permed, straightened or relaxed differently even if they are treated in the same manner. For example, hair may be waved or poorly straightened when there is more hair damage more than expected. If the hair is permed or straightened strongly, the hair may be excessively waved or straightened. Adjustment of how well hair is to be permed or straightened often depends on sensory skills such as the intuition of the practitioner, and waving of hair and/or straightening of frizzled hair as desired is not necessarily easy.

In particular, in the case of long hair, hair ends are likely to stand up and spread out. To prevent this and put the hair ends in order, a treatment for waving or straightening only the hair ends may be performed. However, damage often accumulates at hair ends due to irradiation with sunlight over an extended period of time, and repeated permanent waving treatments and dyeing treatments, and hair ends may not be sufficiently waved or straightened using an ordinary perming treatment.

To avoid such an inconvenience, a technique as described in Patent Document 1, for example, has been reported in which hair damage is suppressed by using, as an oxidizing agent in the second agent, a moderately reactive substance such as nano-bubble water. However, it is desired to develop a hair cosmetic that can be used as a permanent waving agent or a hair straightening agent capable of favorably waving various types of hair, regardless of whether or not the hair is damaged.

In addition, hairstyling agent compositions having various properties, such as liquid, gel, cream, and solid compositions, are known to provide simple methods or means for setting hairstyles.

Conventionally, for hairstyling agent compositions, for example, film-forming polymers and materials that are solid at room temperature (e.g., wax, fat, hydrocarbon oil, etc.) are widely used as hairstyling components for enabling hairstyling, which is a main function of hairstyling agent compositions.

However, when hairstyling is performed using hairstyling agent compositions in which such conventional hairstyling components are used, there are cases where a set hairstyle is deformed by moisture, and the hairstyling agent compositions lack in moisture resistance. Therefore, there is a need for a hairstyling agent composition that provides excellent moisture resistance to a set hairstyle, and that has excellent hairstyle retainability.

Related Art Documents

Patent Document

Patent Document 1: Japanese Laid-Open Patent Publication No. 2013-124250

Summary of Invention

Problems to be Solved by the Invention

The present invention is intended to solve the above-described problems, and an object thereof is to provide a hair cosmetic that can be applied to various types of hair regardless of whether or not the hair is damaged, and a method for producing the same, as well as a method for improving hair quality and a method for setting a hairstyle using the hair cosmetic.

Means for Solving the Problem

The present invention is a hair cosmetic comprising, as an active ingredient, a composite of an amino thiol and an organic acid, wherein the amino thiol is at least one compound selected from the group consisting of cysteamine and a salt thereof, and the organic acid is at least one compound selected from the group consisting of fumaric acid, fumarate, maleic acid, and maleate.

In one embodiment, the composite is a reaction product obtained by reacting 1 mole of the amino thiol with 0.8 to 1.2 moles of the organic acid salt.

In one embodiment, the composite has a reducing power as determined using an iodometry method, of 0.1 mL or less.

In one embodiment, the hair cosmetic is a hairstyle forming agent.

The present invention is also a method for producing a hair cosmetic, comprising heating a mixture of an amino thiol and an organic acid to obtain a composite, wherein the amino thiol is at least one compound selected from the group consisting of cysteamine and a salt thereof, and the organic acid is at least one compound selected from the group consisting of fumaric acid, fumarate, maleic acid, and maleate.

In one embodiment, the heating step is performed at a temperature from 75 to 100°C.

In one embodiment, the mixture is in a form of an aqueous solution containing the amino thiol and the organic acid.

The present invention is also a method for improving hair quality, comprising applying the hair cosmetic to the hair.

In one embodiment, the method further comprises heating the hair after the applying of the hair cosmetic.

The present invention is also a method for forming a hairstyle, comprising applying the hair cosmetic to hair for which the formation of a hairstyle is desired.

In one embodiment, the method comprises heating the hair after the applying of the hair cosmetic.

Effects of Invention

According to the present invention, an amino thiol and an organic acid that have been conventionally known as cosmetic materials are contained as constituent components, and it is therefore possible to provide a hair cosmetic that ensures improved safety for users. When the hair cosmetic according to the present invention is used, for example, as a hairstyle forming agent, it is possible to soften the treated hair, and reduce the torsion and the unevenness of the surface thereof, thus enabling the formation of waves with excellent durability.

Description of Embodiments

(Hair Cosmetic)

The hair cosmetic according to the present invention contains, as an active ingredient, a composite of an amino thiol and an organic acid.

Here, the term "hair cosmetic" as used herein refers to all products intended for improvement of human hair qualities, including, for example, shape, feel, and color. Examples of the hair cosmetic include hairstyle forming agents; hairstyling agents such as hair oil, styling agents, hair finishing sticks, pomade, hair cream, hair solid, hair spray, hair lacquer, hair liquid, hair water, hair wax, hair foam, and hair gel; hair restorers such as hair tonic, hair lotion, hair treatment, hair conditioner, and hair packs; scalp agents such as scalp treatment; hair colorants such as dyeing agents, hair color spray, hair color sticks, color rinse, and hair manicures; hair washing agents such as shampoo and hair washing powder; and hair rinses such as a rinse. Here, the term "hairstyle forming agent" as used herein refers to all hair treating agents that may be used for applying a so-called permanent (or perm) to hair, and includes, for example, hair treating agents (e.g., a permanent waving agent) that are used for straightening permanent and frizzled hair-straightening, in addition to waving and curling.

The amino thiol according to the present invention refers to an amino thiol compound having a simpler structure from among compounds having, in one molecule, an amino group ($-NH_2$) and a thiol group or a sulfhydryl group ($-SH$). Examples of the amino thiol include cysteamine and salts thereof (e.g., cysteamine hydrochloride), and combinations thereof. Such cysteamine has been conventionally used as a reducing agent for permanent waving.

The organic acid according to the present invention can be used as a cosmetic material, and examples thereof include fumaric acid, maleic acid and salts thereof (i.e., fumarate and maleate), and combinations thereof. Examples of the salt constituting the organic acid include sodium salts, potassium salts, magnesium salts, calcium salts, and ammonium salts. When the hair cosmetic according to the present invention is used, for example, as a permanent waving agent, such an organic acid imparts favorable durability to the formed waves, and can make the hair particularly soft.

Fumaric acid and fumarate, as well as combinations thereof are particularly preferable in that wave durability is achieved and hair can be made even softer.

The composite constituting the hair cosmetic according to the present invention includes a reaction product of the above-described amino thiol with the above-described organic acid.

The composite according to the present invention has several features.

For example, the composite is in the form of a liquid at room temperature. Therefore, the composite is advantageous in that the composite is highly fluid and can be easily handled as a hair cosmetic component.

The composite is also water-soluble. Therefore, the composite can be prepared in the form of an aqueous solution, using water (e.g., pure water, distilled water, ion exchanged water, RO water, and tap water), which can further enhance the applicability of the composite to a hair cosmetic.

The composite is a reaction product obtained by reacting 1 mole of the above-described amino thiol with preferably 0.8 to 1.2 moles, more preferably 0.9 to 1.1 moles, and even more preferably 0.95 to 1.05 moles of the above-described organic acid salt. In one embodiment, the composite is a reaction product obtained by reacting approximately equimolar proportions of the above-described amino thiol with the above-described organic acid salt.

Furthermore, the composite according to the present invention does not have a predetermined reducing power and, in this respect, can be clearly distinguished from a reducing agent for hair cosmetics that uses an amino thiol and an organic acid as the raw materials. In one embodiment, the composite according to the present invention has a reducing power as determined using an iodometry method, of preferably 0.1 mL or less, more preferably 0.09 mL or less, and even more preferably 0.06 mL or less. Note that the "reducing power as determined using an iodometry method" can be confirmed as a titer (mL) per gram of a substance to be measured, wherein the titer is obtained by accurately weighing the substance to be measured, and acidifying the substance with sulfuric acid, followed by titration with a 0.05 mol/L iodine solution, using a starch test solution as an indicator.

The above-described composite can be obtained by heating a mixture of the above-described amino thiol and the above-described organic acid. For heating of such a mixture, it is preferable that the mixture is in the form of an aqueous solution containing an amino thiol and an organic acid in that the production efficiency of the composite can be increased. Such a mixture can be produced, for example, either by adding an organic acid to an aqueous amino thiol solution prepared in advance, adding an amino thiol to an aqueous organic acid solution prepared in advance, mixing an aqueous amino thiol solution prepared in advance and an aqueous organic acid solution prepared in advance, or mixing an amino thiol, an organic acid, and water.

The content of the organic acid that may be used to obtain the composite is preferably 0.1 to 300 parts by mass, and more preferably 0.4 to 200 parts by mass, per 100 parts by mass of the amino thiol. When the organic acid content is less than 0.1 parts by mass, the effect of improving the hair quality may not be sufficiently achieved. When the organic acid content exceeds 300 parts by mass, the content ratio of the amino thiol constituting the composite is relatively reduced to become insufficient, as a result of which a portion of the organic acid may remain undissolved. There is no particular limitation on the mixing of the amino thiol and the organic acid, and the mixing may be performed using any means known to those skilled in the art.

The temperature applied to heat the mixture of the amino thiol and the organic acid is not necessarily limited, but is preferably 75 to 100°C, and more preferably 80 to 95°C. When the temperature applied to the mixture is less than 75°C, particles of the organic acid may not dissolve properly in the amino thiol, thus making it difficult to obtain a uniform composite. When the mixture is heated in the form of an aqueous solution as described above, it is practically difficult for the temperature applied for heating to exceed 100°C.

As a result of adding the above-described composite as an active ingredient, the hair cosmetic according to the present invention has a pH of preferably 4 to 12, and more preferably 5 to 10. When the pH is less than below 4, the hair cosmetic may cause hair constriction immediately after application. When the pH exceeds 12, alkali may damage the hair. The hair cosmetic according to the present invention can be adjusted to a pH in the above-described range, for example, by adding an alkaline agent. Examples of the alkaline agent include monoethanolamine, triethanolamine, monoisopropanolamine, amino methyl propanol, arginine, and ammonia, as well as combinations thereof. The alkaline agent may be used in combination with, for example, a predetermined amount of water (e.g., pure water, distilled water, ion exchanged water, RO water, and tap water). In one embodiment of the present invention, the alkaline agent is combined in the form of an aqueous solution with the above-described composite.

The hair cosmetic according to the present invention may contain any additive such as a hairstyle forming agent that may be used for conventional hair cosmetics. Examples of such an additive include a base, a humectant, an antistatic agent, a solubilizing agent, a preservative, a colorant, and a perfuming agent, as well as combinations thereof. The content of the additive that may be added to the hair cosmetic according to the present invention is not particularly limited, and a suitable addition amount may be selected by those skilled in the art.

Since the amino thiol and the organic acid constituting the composite are both well-known as cosmetic materials, the hair cosmetic according to the present invention ensures safety for the hair and the scalp, and can be used by the user without concern regarding safety. Furthermore, the above-described composite can be easily produced by heating a mixture of an amino thiol and an organic acid at a predetermined temperature, and therefore the hair cosmetic according to the present invention also has excellent industrial productivity.

(Method for Improving Hair Quality)

The hair cosmetic according to the present invention can be used to improve the quality of human hair, for example.

Hair quality is improved by applying the above-described hair cosmetic to hair.

Although the application means is not particularly limited, the application is achieved, for example, by spraying of a solution (e.g., an aqueous solution) containing the hair cosmetic, immersion in the solution, or application of the solution either using a hand or a brush. The amount of the hair cosmetic applied to the hair is not particularly limited. For example, an amount suitable for the hair condition, age, sex, or the like of the user may be selected as appropriate.

After applying the hair cosmetic according to the present invention to hair, the cosmetic is preferably left as is for a predetermined period of time in order for the cosmetic to sufficiently seep into the hair. Such a time period is also not necessarily limited, but is preferably 3 to 20 minutes, for example.

Furthermore, the hair to which the above-described hair cosmetic has been applied may be left at room temperature (e.g., 15 to 25°C), or may be heated as needed. For such heating, a hair dryer or a hair iron may be used, for example. The heating temperature is also not necessarily limited, but is 40 to 180°C, for example.

Then, the hair is washed with clean water, and heated as needed using any means (e.g., a hair dryer) known to those skilled in the art.

In this way, hair quality can be improved using the hair cosmetic according to the present invention. With the hair cosmetic according to the present invention, it is possible to improve hair qualities such as hair softness, the presence or absence of torsion, the surface condition (roughness due to irregularities), shine, and the like.

(Method for Forming Hairstyle of Hair)

The hair cosmetic according to the present invention may be used as a hairstyle forming agent for forming a human hairstyle, for example.

Formation of a hairstyle is achieved by applying the above-described hair cosmetic to hair for which the formation of a hairstyle is desired.

Although the application means is not particularly limited, application is achieved, for example, by spraying of a solution (e.g., an aqueous solution) containing the hair cosmetic, immersion in the solution, or application of the solution either using a hand or a brush. The amount of the hair cosmetic applied to hair is not particularly limited. For example, an amount suitable for the hair condition, age, sex, or the like of the user may be selected as appropriate.

After applying the hair cosmetic according to the present invention to hair, the cosmetic is preferably left as is for a predetermined period of time in order for the cosmetic to sufficiently seep into the hair. Such a time period is also not necessarily limited, but is preferably 3 to 20 minutes, for example.

Furthermore, the hair to which the above-described hair cosmetic has been applied may be left at room temperature (e.g., 15 to 25°C), or may be heated as needed. For such heating, an accelerator, a hair steamer, a hair dryer, or a hair iron may be used, for example. The heating temperature is also not necessarily limited, but is 40 to 180°C, for example.

When the hair cosmetic according to the present invention is used as a one-part hairstyle forming agent, the hair is then washed with clean water, and heated as needed using any means (e.g., a hair dryer) known to those skilled in the art.

In this way, the hairstyle can be formed in the desired form, using the hair cosmetic according to the present invention.

Examples

The present invention will be described in greater detail below by way of examples. However, the present invention is not limited to these examples.

(Example 1: Production of Aqueous Hair Cosmetic Solution (E1) Using Cysteamine and Fumaric Acid)

Cysteamine (prepared in the form of an aqueous solution in advance) in an amount of 100 parts by mass and fumaric acid in an amount of 150 parts by mass were mixed, and the mixture was heated at 95°C to allow cysteamine to react with fumaric acid, thereby obtaining an aqueous solution containing a composite of cysteamine and fumaric acid. The aqueous solution of the composite was adjusted using water such that the composite had a concentration (concentration based on the total amount of cysteamine and fumaric acid) of 5 % by mass and a pH of 8.2, thereby obtaining an aqueous hair cosmetic solution (E1).

The aqueous hair cosmetic solution (E1) was accurately weighed and acidified with sulfuric acid, followed by titration (iodometry method) with a 0.05 mol/L iodine solution, using a starch test solution as an indicator, thereby measuring the reducing power (mL) of the aqueous hair cosmetic solution (E1). The reducing power was less than or equal to 0.02 mL, which was the detection limit, and the obtained composite was confirmed to have resulted from a reaction between approximately equimolar amounts of cysteamine and fumaric acid.

(Example 2: Production of Aqueous Hair Cosmetic Solution (E2) Using Cysteamine and Maleic acid)

An aqueous hair cosmetic solution (E2) containing a composite of cysteamine and maleic acid was obtained in the same manner as in Example 1 except that 150 parts by mass of maleic acid was used in place of fumaric acid.

For the aqueous hair cosmetic solution (E2), the reducing power (mL) as determined using an iodometry method was measured in the same manner as in Example 1. The reducing power was 0.02 mL or less, which was the detection limit, and the obtained composite was confirmed to have resulted from a reaction between of approximately equimolar amounts of cysteamine and maleic acid.

(Comparative Example 1: Production of Aqueous Hair Cosmetic Solution (C1) Using Cysteamine and Succinic Acid)

An aqueous hair cosmetic solution (C1) containing a composite of cysteamine and succinic acid was obtained in the same manner as in Example 1 except that 153 parts by mass of succinic acid was used in place of fumaric acid.

For the aqueous hair cosmetic solution (C1), the reducing power (mL) as determined using an iodometry method was measured in the same manner as in Example 1. The reducing power was 2.57 mL.

(Comparative Example 2: Production of Aqueous Hair Cosmetic Solution (C2) Using Cysteamine and Levulinic acid)

An aqueous hair cosmetic solution (C2) containing a composite of cysteamine and levulinic acid was obtained in the same manner as in Example 1 except that 150 parts by mass of levulinic acid was used in place of fumaric acid.

(Comparative Example 3: Production of Aqueous Hair Cosmetic Solution (C3) Using Cysteamine and Malic acid)

An aqueous hair cosmetic solution (C3) containing a composite of cysteamine and malic acid was obtained in the same manner as in Example 1 except that 174 parts by mass of malic acid was used in place of fumaric acid.

(Comparative Example 4: Production of Aqueous Hair Cosmetic Solution (C4) Using Cysteamine and Ketoglutaric Acid)

An aqueous hair cosmetic solution (C4) containing a composite of cysteamine and ketoglutaric acid was obtained in the same manner as Example 1 except that 189 parts by mass of ketoglutaric acid was used in place of fumaric acid.

(Comparative Example 5: Production of Aqueous Hair Cosmetic Solution (C5) Using Cysteamine and Glyoxylic Acid)

An aqueous hair cosmetic solution (C5) containing a composite of cysteamine and glyoxylic acid was obtained in the same manner as in Example 1 except that 96 parts by mass of glyoxylic acid was used in place of fumaric acid.

(Comparative Example 6: Production of Aqueous Hair Cosmetic Solution (C6) Using Cysteamine and Citric Acid)

An aqueous hair cosmetic solution (C6) containing a composite of cysteamine and citric acid was obtained in the same manner as Example 1 except that 249 parts by mass of citric acid was used in place of fumaric acid.

(Comparative Example 7: Production of Hair Cosmetic (C7) Using Cysteine and Fumaric Acid)

An aqueous hair cosmetic solution (C7) containing a composite of cysteine and fumaric acid was obtained in the same manner as in Example 1 except that 157 parts by mass of cysteine was used in place of cysteamine, and that adjustment was performed such that the composite had a concentration (concentration based on the total amount of cysteine and fumaric acid) of 8 % by mass and a pH of 8.0.

(Comparative Example 8: Production of Hair Cosmetic (C8) Using Glyceryl Monothioglycolate and Fumaric Acid)

An aqueous hair cosmetic solution (C8) containing a composite of glyceryl monothioglycolate (GMT) and fumaric acid was obtained in the same manner as in Example 1 except that 216 parts by mass of GMT was used in place of cysteamine, and that adjustment was performed such that the composite had a concentration (concentration based on the total amount of GMT and fumaric acid) of 5 % by mass and a pH of 7.0.

(Comparative Example 9: Production of Hair Cosmetic (C9) Using Glutathione and Fumaric Acid)

An aqueous hair cosmetic solution (C9) containing a composite of glutathione and fumaric acid was obtained in the same manner as in Example 1 except that 398 parts by mass of glutathione was used in place of cysteamine, and that adjustment was performed such that the composite had a concentration (concentration based on the total amount of glutathione and fumaric acid) of 8 % by mass and a pH of 9.0.

(Comparative Example 10: Production of Hair Cosmetic (C10) Using Spiera and Fumaric Acid)

An aqueous hair cosmetic solution (C10) containing a composite of Spiera and fumaric acid was obtained in the same manner as in Example 1 except that 153 parts by mass of Spiera (butyrolactonethiol) was used in place of cysteamine, and that adjustment was performed such that the composite had a concentration (concentration based on the total amount of Spiera and fumaric acid) of 1 % by mass and a pH of 6.0.

(Comparative Example 11: Production of Hair Cosmetic (C11) Using Sodium Sulfite and Fumaric Acid)

An aqueous hair cosmetic solution (C11) containing a composite of sodium sulfite and fumaric acid was obtained in the same manner as in Example 1 except that 164 parts by mass of sodium sulfite was used in place of cysteamine, and that adjustment was performed such that the composite had a concentration (concentration based on the total amount of cysteine and fumaric acid) of 8 % by mass and a pH of 9.0.

(Evaluation of Waving Ability)

Bundles each including 20 human hairs (average length: 15 cm) were wound in a zigzag pattern on plastic measurement combs, fixed to the measurement combs, and then treated by being immersed for 10 minutes in a perming solution (pH 9.5) of 10 % by mass of ammonium thioglycolate, and thereafter rinsed for 1 minute with running water at 40°C, according to a Kirby method (Proceedings of the Scientific Section Volume 26, page 12, 1956). Then, the treated hair bundles were divided into three parts so as to have substantially the same amounts, and each of the three parts was immersed in each of the aqueous hair cosmetic solutions (E1) and (E2) produced in Examples 1 and 2, as well as the hair cosmetics (C1) to (C11) produced in Comparative Examples 1 to 11 for 1 minute, and thereafter was taken out.
Thereafter, the hair bundles still fixed to these measurement combs were subjected to one of the following treatments: (1) a room-temperature treatment, (2) a medium-temperature treatment, and (3) a high-temperature treatment.

(1) Room-Temperature Treatment (Kirby method)

Each of the hair bundles still fixed to the measurement combs taken out from each of the aqueous hair cosmetic solutions was left for 10 minutes at room temperature of 15 to 20°C, and thereafter immersed for 10 minutes in a second solution constituted by a 6 % by mass aqueous sodium bromate solution. After being taken out from the second solution, each hair bundle was removed from the measurement comb, and dried at room temperature.
Once dried, the treated hair bundles were subjected to the following evaluations.

(Curl Feel)

Each of the hair bundles treated as described above was touched by the hand of an expert in the field, and the obtained tactile impression was compared with that obtained when a 10 % by mass aqueous ammonium thioglycolate solution (pH 9.2; a first solution) and an aqueous sodium bromate solution (a second solution), which are perming solutions for a commonly used 2nd agent for permanent wave, were used in place of each of the aqueous hair cosmetic solutions (when the first solution was left for 10 minutes after being applied, and thereafter the second solution was applied), and the result was evaluated according to the following criteria.
Score 4 Excellent: Both the firmness and the tactile feel of curls were significantly enhanced.

Score 3 Good: Both the firmness and the tactile feel of curls were enhanced, with one of the firmness and the tactile feel in particular being significantly enhanced.

Score 2 Slightly effective: One of the firmness and the tactile feel of curls was enhanced.

Score 1 Little effect: Both the firmness and the tactile feel of curls were not significantly different from those obtained when the aqueous ammonium thioglycolate solution was used.

Score 0: Both the firmness and the tactile feel of curls deteriorated as compared with those obtained when the aqueous ammonium thioglycolate solution was used.

(Curl Durability)

Each of the hair bundles treated as described above was immersed for 1 minute at room temperature in a solution obtained by diluting a commercially available shampoo 10 times by volume with water, thereafter rinsed with warm water at 38°C for 30 seconds, dried with a towel, and further dried with a hair dryer. The state of curls in the thus obtained hair bundle was visually compared by an expert in the field with that obtained when a 10 % by mass aqueous ammonium thioglycolate solution (pH 9.2; a first solution) and an aqueous sodium bromate solution (a second solution), which are perming solutions for a commonly used 2nd agent for permanent wave, were used in place of each of the aqueous hair cosmetic solutions (when the first solution was left for 10 minutes after being applied, and thereafter the second solution was applied), and the result was evaluated according to the following criteria.

Score 4 Excellent overall: The state of curls remained unchanged even after the above-described treatment using the shampoo had been performed 30 times or more.

Score 3 Good overall: The state of curls changed after the above-described treatment using the shampoo had been performed 20 times or more and 29 times or less.

Score 2 Slightly effective overall: The state of curls changed after the above-described treatment using the shampoo had been performed 10 times or more and 19 times or less.

Score 1 Little effect overall: The state of curls changed after the above-described treatment using the shampoo had been performed 1 time or more and 9 times or less.

Score 0: The state of curls deteriorated as compared with that obtained when the aqueous ammonium thioglycolate solution was used, even before the above-described treatment using the shampoo had been performed.

The obtained results are shown in Table 1.

[Table 1]

| Room-temperature treatment (15°C to 20°C) | | | | | Evaluation of curls | | |
|---|---|---|---|---|---|---|---|
| | Aqueous hair cosmetic solution | Reducing agent | Organic acid | Reducing power as determined using iodometry method | Feel (score) | Durability (score) | Remarks |
| Example 1 | (E1) | Cysteamine | Fumaric acid | 0.02 mL or less | 3 | 2 | Formed curls were loose. |
| Example 2 | (E2) | Cysteamine | Maleic acid | 0.02 mL or less | 3 | 2 | Formed curls were loose. |
| Comparative Example 1 | (C1) | Cysteamine | Succinic acid | 2.57 mL | 2 | 3 | Formed curls retained to a certain degree. |
| Comparative Example 2 | (C2) | Cysteamine | Levulinic acid | - 1) | 3 | 1 | Curls were formed, but were uncurled after the fifth shampooing. |

(continued)

| Room-temperature treatment (15°C to 20°C) | | | | | | | |
|---|---|---|---|---|---|---|---|
| | Aqueous hair cosmetic solution | Reducing agent | Organic acid | Reducing power as determined using iodometry method | Evaluation of curls | | |
| | | | | | Feel (score) | Durability (score) | Remarks |
| Comparative Example 3 | (C3) | Cysteamine | Malic acid | - | 3 | 1 | Curls were formed, but were uncurled after the third shampooing. |
| Comparative Example 4 | (C4) | Cysteamine | Ketoglutaric acid | - | 2 | 1 | Formed curls were loose. |
| Comparative Example 5 | (C5) | Cysteamine | Glyoxylic acid | - | 1 | 0 | Curls collapsed. |
| Comparative Example 6 | (C6) | Cysteamine | Citric acid | - | 1 | 1 | Curls were loose, and were immediately uncurled after the first shampooing. |
| 1) Not measured | | | | | | | |

(2) Medium-Temperature Treatment (Kirby method)

Each of the hair bundles still fixed to the measurement combs taken out from each of the aqueous hair cosmetic solutions was left for 7 minutes at a room temperature of 15 to 20°C, and thereafter exposed to hot air at about 80°C for 3 minutes using a hair dryer. Thereafter, the hair bundle was immersed for 10 minutes in a second solution constituted by a 6 % by mass aqueous sodium bromate solution. After being taken out from the second solution, the hair bundle was removed from the measurement comb, and dried at room temperature. Once dried, for the treated hair, the curl feel and the curl durability were evaluated in the same manner as described above. The obtained results are shown in Table 2.

[Table 2]

| Medium-temperature treatment (about 70°C) | | | | | | | |
|---|---|---|---|---|---|---|---|
| | Aqueous hair cosmetic solution | Reducing agent | Organic acid | Reducing power as determined using iodometry method | Evaluation of curls | | |
| | | | | | Feel (score) | Durability (score) | Remarks |
| Example 1 | (E1) | Cysteamine | Fumaric acid | 0.02 mL or less | 3 | 2 | Formed curls retained, with an excellent tactile feel. |
| Example 2 | (E2) | Cysteamine | Maleic acid | 0.02 mL or less | 2 | 2 | Formed curls retained, with a good tactile feel. |

(continued)

| Medium-temperature treatment (about 70°C) | | | | | | | |
| | Aqueous hair cosmetic solution | Reducing agent | Organic acid | Reducing power as determined using iodometry method | Evaluation of curls | | |
| | | | | | Feel (score) | Durability (score) | Remarks |
| Comparative Example 1 | (C1) | Cysteamine | Succinic acid | 2.57 mL | 2 | 3 | Formed curls retained, with good firmness. |
| Comparative Example 2 | (C2) | Cysteamine | Levulinic acid | - 1) | 2 | 2 | Curls were formed, but were uncurled after shampooing 20 times or less. |
| Comparative Example 3 | (C3) | Cysteamine | Malic acid | - | 1 | 1 | Curls were formed, but were uncurled after the fifth shampooing. |
| Comparative Example 4 | (C4) | Cysteamine | Ketoglutaric acid | - | 2 | 2 | Formed curls retained, but the curls stiffened. |
| Comparative Example 5 | (C5) | Cysteamine | Glyoxylic acid | - | 1 | 0 | No curls were formed. |
| Comparative Example 6 | (C6) | Cysteamine | Citric acid | - | 1 | 1 | Curls were formed, but were immediately uncurled after the first shampooing. |
| 1) Not measured | | | | | | | |

(3) High-Temperature Treatment

Twenty human hairs (average length: 15 cm) were treated by being immersed for 10 minutes in a perming solution (pH 9.5) of 10 % by mass of ammonium thioglycolate, and thereafter being rinsed for 1 minute with running water at 40°C. Then, the treated hairs were immersed for 1 minute in each of the aqueous hair cosmetic solutions (E1) and (E2) produced in Examples 1 and 2, and the hair cosmetics (C1) to (C6) produced in Comparative Examples 1 to 6, and thereafter taken out. Excess water was removed from the hairs taken out from each of the aqueous hair cosmetic solutions, and the hair was then wound around and an 18-mm hair iron (about 180°C) twice, and left for 5 minutes to curl. Thereafter, the hairs were removed from the hair iron, and left for 5 minutes at room temperature. Thereafter, the hairs were immersed for 10 minutes in a second solution constituted by a 6 % by mass aqueous sodium bromate solution, and taken out therefrom, followed by drying at room temperature. Once dried, the curl feel and the curl durability of the treated hairs were evaluated in the same manner as describe above. The obtained results are shown in Table 3.

[Table 3]

| High-temperature treatment (about 180°C) | | | | | | |
| --- | --- | --- | --- | --- | --- | --- |
| | Aqueous hair cosmetic solution | Reducing agent | Organic acid | Reducing power as determined using iodometry method | Evaluation of curls | | |
| | | | | | Feel (score) | Durability (score) | Remarks |
| Example 1 | (E1) | Cysteamine | Fumaric acid | 0.02 mL or less | 4 | 4 | Curls that were soft and free of torsion and that had reduced irregularities and excellent durability were formed. |
| Example 2 | (E2) | Cysteamine | Maleic acid | 0.02 mL or less | 2 | 4 | Curls that were soft and that had few residual irregularities and excellent durability were formed. |
| Comparative Example 1 | (C1) | Cysteamine | Succinic acid | 2.57 mL | 2 | 3 | The hairs stiffened, and curls were formed, with a certain degree of durability confirmed. |
| Comparative Example 2 | (C2) | Cysteamine | Levulinic acid | - [1] | 2 | 3 | The hairs stiffened, and curls were formed, but lacked durability in the subsequent shampooing. |
| Comparative Example 3 | (C3) | Cysteamine | Malic acid | - | 1 | 2 | The hairs stiffened, and curls were formed, but clearly lacked durability. |
| Comparative Example 4 | (C4) | Cysteamine | Ketoglutaric acid | - | 2 | 2 | The hairs stiffened, and curls were difficult to form. |

(continued)

| High-temperature treatment (about 180°C) | | | | | | | |
| --- | --- | --- | --- | --- | --- | --- | --- |
| | Aqueous hair cosmetic solution | Reducing agent | Organic acid | Reducing power as determined using iodometry method | Evaluation of curls | | |
| | | | | | Feel (score) | Durability (score) | Remarks |
| Comparative Example 5 | (C5) | Cysteamine | Glyoxylic acid | - | 0 | 0 | Curls collapsed, and were rough. |
| Comparative Example 6 | (C6) | Cysteamine | Citric acid | - | 1 | 1 | The hairs stiffened, and no curls were formed. |
| 1) Not measured | | | | | | | |

As can be clearly seen from the results shown in Tables 1 to 3, in the case of the room-temperature treatment, the aqueous hair cosmetic solutions (E1) and (E2) obtained in Examples 1 and 2 each provided an excellent curl feel superior to those provided by the aqueous hair cosmetic solutions (C1) to (C6) obtained in Comparative Examples 1 to 6. Furthermore, it can also be seen that, in the case of the high-temperature treatment as well, the aqueous hair cosmetic solution (E1) obtained in Example 1 provided excellent curl feel and durability superior to those provided by the aqueous hair cosmetic solutions obtained in the other examples and the comparative examples.

(Evaluation of Hair Quality Improvement)

Monoethanolamine was added to test solutions of the aqueous hair cosmetic solutions (E1) and (C7) to (C11) produced in Example 1 and Comparative Examples 7 to 11 to adjust the pHs thereof, and the content of the reducing agent and the pH of each of the test solutions obtained thereafter are shown in Table 4.
Then, 0.2 g (average length: 15 cm) of damaged human hairs were immersed for 10 minutes in each of the test solutions, and thereafter rinsed for 1 minute with running water at 40°C, and then dried using a hair dryer. Once dried, the hairs were slowly passed through a straightening iron at 180°C, rinsed again for 1 minute with running water, and dried using a hair dryer. The feel of the treated hairs was evaluated in the same manner as described above. The obtained results are shown in Table 4.

[Table 4]

| High-temperature treatment (about 180°C) | | | | | | | | |
|---|---|---|---|---|---|---|---|---|
| | Aqueous hair cosmetic solution | Reducing agent | Organic acid | Reducing power as determined using iodometry method | Composite content (mass%) | pH | Evaluation of curls | |
| | | | | | | | Feel (score) | Remarks |
| Example 1 | (E1) | Cysteamine | Fumaric acid | 0.02 mL or less | 5 | 8.2 | 3 | Although the hair had been reinforced, few soft portions were formed therein. |
| Comparative Example 7 | (C7) | Cysteine | Fumaric acid | - 1) | 8 | 8.0 | 1 | The hair stiffened. |
| Comparative Example 8 | (C8) | GMT | Fumaric acid | - | 5 | 7.0 | 2 | Although few soft portions were formed, the hair had an unpleasant odor. |
| Comparative Example 9 | (C9) | Glutathione | Fumaric acid | - | 8 | 9.0 | 1 | No effect was observed. |
| Comparative Example 10 | (C10) | Spiera | Fumaric acid | - | 1 | 6.0 | 0 | The composite was not formed properly. |
| Comparative Example 11 | (C11) | Sodium sulfite | Fumaric acid | - | 8 | 9.0 | 1 | The hair seemed to be unkempt. |
| 1) Not measured | | | | | | | | |

13

As shown in Table 4, it can be seen that aqueous hair cosmetic solution (E1) obtained in Example 1 was superior to the aqueous hair cosmetic solutions (C7) to (C11) obtained in Comparative Examples 7 to 11 in that the hairs did not have an unpleasant odor, and the damaged portions of the hairs were softened and reinforced.

(Example 3: Production of Hair-Waving Intermediate Treatment Agent (J1))

Cysteamine (prepared in the form of an aqueous solution in advance) in an amount of 100 parts by mass and fumaric acid in an amount of 150 parts by mass were mixed, and the mixture was heated at 95°C to allow cysteamine to react with fumaric acid. Thereafter, ammonia water was added to the resulting solution to adjust the pH thereof to 7.0, thus obtaining an aqueous solution containing a composite of cysteamine and fumaric acid (this aqueous solution is referred to as a stock solution 1 of the intermediate treatment agent). RO water was added to the aqueous solution of the composite, thereby obtaining a hair-waving intermediate treatment agent (J1) in which the composite had a concentration (concentration based on the total amount of cysteamine and fumaric acid) of 0.5 % by mass.

For the hair-waving intermediate treatment agent (J1), the reducing power (mL) as determined using an iodometry method was measured in the same manner as in Example 1. The reducing power was less than or equal to 0.02 mL, which was the detection limit, and the obtained composite was confirmed to have resulted from a reaction between approximately equimolar amounts of cysteamine and fumaric acid.

(Example 4: Production of Hair-Waving Intermediate Treatment Agent (J2))

An aqueous solution (pH7.0) containing a composite of cysteamine and maleic acid was obtained (this aqueous solution is referred to as a stock solution 2 of the intermediate treatment agent) in the same manner as in Example 3 except that 150 parts by mass of maleic acid was used in place of fumaric acid. RO water was added to the aqueous solution of the composite, thereby obtaining a hair-waving intermediate treatment agent (J2) in which the composite had a concentration (concentration based on the total amount of cysteamine and maleic acid) of 0.5 % by mass.

For the hair-waving intermediate treatment agent (J2), the reducing power (mL) as determined using an iodometry method was measured in the same manner as in Example 1. The reducing power was less than or equal to 0.02 mL, which was the detection limit, and the obtained composite was confirmed to have resulted from a reaction between approximately equimolar amounts cysteamine and maleic acid.

(Comparative Example 12: Production of Hair-Waving Intermediate Treatment Agent (H1))

An aqueous solution (pH7.0) containing a composite of cysteamine and maleic anhydride was obtained (this aqueous solution is referred to as a stock solution 3 of the intermediate treatment agent) in the same manner as in Example 3 except that 127.1 parts by mass of maleic anhydride was used in place of fumaric acid. RO water was added to the aqueous solution of the composite, thereby obtaining a hair-waving intermediate treatment agent (H1) in which the composite had a concentration (concentration based on the total amount of cysteamine and maleic anhydride) of 0.46 % by mass.

For the hair-waving intermediate treatment agent (H1), the reducing power (mL) as determined using an iodometry method was measured in the same manner as in Example 1. The reducing power was less than or equal to 0.02 mL, which was the detection limit.

(Production of Waved Hairs)

RO water was added to a mixture of a 50 % by mass aqueous ammonium thioglycolate solution and an 80 % by mass aqueous monoethanolamine solution, to produce a first agent for permanent wave (hereinafter referred to as a "first agent") having a thioglycolic acid concentration of 10 % by mass and a pH of 9.0. RO water was added to sodium bromate to produce a second agent for permanent wave (hereinafter referred to as a "second agent") having a sodium bromate concentration of 6 % by mass.

Using the first agent and the second agent obtained in this manner, as well as the intermediate treatment agents (J1), (J2), and (H1) obtained in the Example 3, Example 4, and Comparative Example 12, the following waving treatment was performed on hair bundles composed of human hairs.

First, the length (average value) of each of the hair bundles before being treated was measured, and the hair bundle was immersed in the first agent for 10 minutes at room temperature. Then, the hair bundle taken out from the first solution was immersed in either the intermediate treatment agent (J1), (J2) or (H1) for 2 minutes at room temperature, and thereafter washed with warm water. The hair bundle was wound around a digital perming rod, and heated for 10 minutes at 80°C, and the hair bundle removed from the rod was immersed in the second agent for 10 minutes at room temperature. After being taken out, the hair bundle was washed with warm water, and dried with warm air. In this manner, the waved hairs (treated hair bundle) were obtained.

(Evaluation of Waving Ability)

The length (average value) of the treated hair bundles (waved hairs) obtained in the above-described manner was measured. From the measured length and the corresponding length measured in advance of the pre-treatment hair bundles, the hair waving ability (%) was calculated according to the following expression.

[Formula 1]

Hair waving ability (%) = [(Length of hair bundle before being treated − Length

of treated hair bundle)/Length of hair bundle before being treated] × 100

The results are shown in Table 5.

[Table 5]

|  | Intermediate treatment agent | Reducing agent | Organic acid | Reducing power as determined using iodometry method | Evaluation of waving ability | | |
|---|---|---|---|---|---|---|---|
|  |  |  |  |  | Length of original hair bundle (cm) | Length of treated hair bundle (cm) | Waving ability (%) |
| Example 3 | (J1) | Cysteamine | Fumaric acid | 0.02 mL or less | 23.7 | 19.8 | 16.5 |
| Example 4 | (J2) | Cysteamine | Maleic acid | 0.02 mL or less | 24.3 | 21.0 | 13.5 |
| Comparative Example 12 | (H1) | Cysteamine | Maleic anhydride | 0.02 mL or less | 24.1 | 21.2 | 12.0 |

As shown in Table 5, the hairs waved respectively using the intermediate treatment agents (J1) and (J2) obtained in Examples 3 and 4 each exhibited a high value of the waving ability as compared with the hairs waved using the intermediate treatment agent (H1) obtained in Comparative Example 12, and it can be seen that the intermediate treatment agents (J1) and (J2) each have an excellent waving ability.
In addition, from the results of Example 4 and Comparative Example 12 shown in Table 5, it can be seen that, for the intermediate treatment agents (J2) and (H1) containing maleic acid (Example 4) or maleic anhydride (Comparative Example 12) as a component of the intermediate treatment agent, the waving ability differs depending on whether or not the component is an anhydride.

(Evaluation of Wave Retaining Ability)

The treated hair bundle (waved hairs) obtained in the above-described manner was subjected to 10 repetitions of washing with a commercially available shampoo and drying with warm air, to obtain a washed hair bundle. The length (average value) of the washed hair bundle was measured. From the measured length and the corresponding length measured in advance of the pre-treatment hair bundle, the post-washing waving ability (%) was calculated according to the following expression.

[Formula 2]

Post-washing waving ability (%) = [(Length of hair bundle before being treated

- Length of washed hair bundle)/Length of hair bundle before being treated] × 100

In addition, from the value of the post-washing waving ability, and the value of the (pre-washing) hair waving ability obtained in Table 5, the hair wave retaining ability (%) against washing was calculated according to the following expression.

[Formula 3]

Hair wave retaining ability against washing (%) = [(Post-washing) hair waving ability (%)/Pre-washing waving ability (%)] × 100

The results are shown in Table 6.

[Table 6]

| | Intermediate treatment agent | Reducing agent | Organic acid | Reducing power as determined using iodometry method | Evaluation of post-washing waving ability | | | Hair wave retaining ability against washing (%) |
| --- | --- | --- | --- | --- | --- | --- | --- | --- |
| | | | | | Length of original hair bundle (cm) | Length of washed hair bundle (cm) | Post-washing waving ability (%) | |
| Example 3 | (J1) | Cysteamine | Fumaric acid | 0.02 mL or less | 23.7 | 20.7 | 12.6 | 76.3 |
| Example 4 | (J2) | Cysteamine | Maleic acid | 0.02 mL or less | 24.3 | 22.5 | 7.4 | 54.8 |
| Comparative Example 12 | (H1) | Cysteamine | Maleic anhydride | 0.02 mL or less | 24.1 | 22.7 | 5.8 | 48.3 |

As shwon in Table 6, the hairs waved respectively using the intermediate treatment agents (J1) and (J2) obtained in Examples 3 and 4 each exhibited a high value of the post-washing waving ability as compared with the hairs waved using the intermediate treatment agent (H1) obtained in Comparative Example 12, and it can be seen that the intermediate treatment agents (J1) and (J2) can provide an excellent waving ability for washed waved hairs as well.

In addition, from the results of Example 4 and Comparative Example 12 shown in Table 6, it can be seen that, for the intermediate treatment agents (J2) and (H1) containing maleic acid (Example 4) or maleic anhydride (Comparative Example 12) as a component of the intermediate treatment agent, the post-washing waving ability differs depending on whether or not the component is an anhydride.

As described above, the intermediate treatment agents (J1) and (J2) of Examples 3 and 4, which used fumaric acid or maleic acid as an organic acid, each had an excellent waving ability both for washed hairs and hairs before being washed as compared with the intermediate treatment agent (H1) of the Comparative Example 12, which used maleic anhydride as an anhydride, and were able to favorably maintain the waving ability even after repeated washing. The effects thereof, in order from the best to the worst, were as follows: Intermediate treatment agents (J1) > (J2) > (H1). In addition, it can be seen that the excellent waving ability is not attributed to the reducing power of the intermediate treatment agent, but is another effect.

(Example 5: Production of Curling Agent (KE1) Using Cysteamine Hydrochloride and Fumaric Acid)

An aqueous solution containing a composite of cysteamine hydrochloride and fumaric acid was prepared in the same manner as in Example 1 except that cysteamine hydrochloride was used in place of cysteamine, and that adjustment was performed such that the concentration of the obtained composite was 12 % by mass. Then, 5 parts by mass of the aqueous solution and 95 parts by mass of water were mixed, thereby obtaining a curling agent (KE1).

For the curling agent (KE1), the reducing power (mL) as determined using an iodometry method was measured in the same manner as in Example 1. The reducing power was less than or equal to 0.02 mL, which was the detection limit, and the obtained composite was confirmed to have resulted from a reaction between approximately equimolar amounts of cysteamine hydrochloride and maleic acid.

(Comparative Example 13: Production of Curling Agent (KC1))

A trace amount of ammonia water was added to a predetermined amount of cysteamine hydrochloride and a predetermined amount of fumaric acid, and the mixture was diluted with water such that the total amount was 100 % by mass, to obtain a curling agent (KC1). That is, the curling agent (KC1) did not contain cysteamine and fumaric acid in the form of a composite as that of Example 5, and had a cysteamine hydrochloride concentration of 0.3 % by mass and a fumaric acid concentration of 0.3 % by mass, separately.

(Evaluation of Curl Keeping Ability)

Bleached hair bundles (bleached hair bundles before being treated) were immersed for 5 minutes in the curling agent (KE1) obtained in Example 5, the curling agent (KC1) obtained in Comparative Example 13, and water (100 % by mass) as a blank, respectively. Then, the bleached hairs of each of the bundles were taken out, rinsed with water at about 40°C, wound around a 15-mm perming rod, and dried for 5 minutes with warm air at about 80°C using a hair dryer. The bleached hairs were removed from the perming rod, and left for 1 hour under the conditions of 40°C and 88% humidity.

For the bleached hair bundles treated in this manner, the Curl keeping ability (%) was calculated according to the following expression.

[Formula 4]

$$\text{Curl keeping ability (\%)} = 100 \times [(\text{Length of bleached hair bundle before being treated} - \text{Length of treated bleached hair bundle}/(\text{Length of bleached hair bundle before being treated})$$

The results are shown in Table 7.

[Table 7]

| | Curling agent | Evaluation of curl keeping ability | | |
|---|---|---|---|---|
| | | Length of hair bundle before being treated (mm) | Length of treated hair bundle (mm) | Curl keeping ability (%) |
| Example 5 | Aqueous solution containing a composite of cysteamine hydrochloride and fumaric acid | 256 | 172 | 32.8 |
| Comparative Example 13 | Aqueous solution containing a simple mixture (non-composite) of cysteamine hydrochloride and fumaric acid | 255 | 218 | 14.5 |
| Blank | Water | 264 | 260 | 1.5 |

As shown in Table 7, it can be seen that the bleached hair bundle treated using the curling agent obtained in Example 5 had an improved hair curl keeping ability as compared with those treated using the curling agent obtained in Comparative Example 13 and the blank, and can provide an excellent curl keeping ability for hair.

INDUSTRIAL APPLICABILITY

The present invention is useful, for example, in the field of cosmetic manufacturing as well as business fields of hairdressing and beauty.

**Claims**

1.  A hair cosmetic comprising, as an active ingredient, a composite of an amino thiol and an organic acid,
    wherein the amino thiol is at least one compound selected from the group consisting of cysteamine and a salt thereof, and the organic acid is at least one compound selected from the group consisting of fumaric acid, fumarate, maleic acid, and maleate.

2.  The hair cosmetic according to claim 1, wherein the composite is a reaction product obtained by reacting 1 mole of the amino thiol with 0.8 to 1.2 moles of the organic acid salt.

3.  The hair cosmetic according to claim 1 or 2, wherein the composite has a reducing power as determined using an iodometry method, of 0.1 mL or less.

4.  The hair cosmetic according to any one of claims 1 to 3, wherein the hair cosmetic is a hairstyle forming agent.

5.  A method for producing a hair cosmetic, comprising heating a mixture of an amino thiol and an organic acid to obtain a composite,
    wherein the amino thiol is at least one compound selected from the group consisting of cysteamine and a salt thereof, and the organic acid is at least one compound selected from the group consisting of fumaric acid, fumarate, maleic acid, and maleate.

6.  The method according to claim 5, wherein the heating step is performed at a temperature from 75 to 100°C.

7.  The method according to claim 5 or 6, wherein the mixture is in a form of an aqueous solution containing the amino thiol and the organic acid.

8.  A method for improving hair quality, comprising
    applying the hair cosmetic according to any one of claims 1 to 4 to the hair.

9.  The method according to claim 8, further comprising heating the hair after the applying of the hair cosmetic.

10. A method for forming a hairstyle, comprising applying the hair cosmetic according to any one of claims 1 to 4 to hair for which the formation of a hairstyle is desired.

**11.** The method according to claim 10, further comprising heating the hair after the applying of the hair cosmetic.

## INTERNATIONAL SEARCH REPORT

| International application No. |
|---|
| **PCT/JP2021/039567** |

**A. CLASSIFICATION OF SUBJECT MATTER**

*A61K 8/362*(2006.01)i; *A61K 8/46*(2006.01)i
FI: A61K8/362; A61K8/46

According to International Patent Classification (IPC) or to both national classification and IPC

**B. FIELDS SEARCHED**

Minimum documentation searched (classification system followed by classification symbols)

A61K8/362; A61K8/46

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 4-243860 A (L'OREAL) 31 August 1992 (1992-08-31)<br>claim 1, paragraphs [0007]-[0008], examples I(b), 1-2, 7-11 | 1-11 |
| Y | JP 2-248410 A (NITTO BOSEKI CO LTD) 04 October 1990 (1990-10-04)<br>claim 1 | 1-11 |
| Y | WO 2009/051005 A1 (NITTO BOSEKI CO LTD) 23 April 2009 (2009-04-23)<br>claims 1-2, paragraph [0042] | 1-11 |
| Y | JP 2004-223122 A (NONOGAWA SHOJI YK) 12 August 2004 (2004-08-12)<br>claims 1-4, paragraph [0004] | 9, 11 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 November 2021** | **21 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/039567**

| Patent document cited in search report | | | Publication date (day/month/year) | Patent family member(s) | Publication date (day/month/year) |
|---|---|---|---|---|---|
| JP | 4-243860 | A | 31 August 1992 | US 5154918 A<br>claim 1, p. 4, left column,<br>lines 1-68, examples I(b), 1-2,<br>7-11<br>EP 465342 A1 | |
| JP | 2-248410 | A | 04 October 1990 | (Family: none) | |
| WO | 2009/051005 | A1 | 23 April 2009 | US 2010/0215940 A1<br>claims 1-2, paragraph [0044]<br>EP 2199265 A1<br>CN 101687698 A | |
| JP | 2004-223122 | A | 12 August 2004 | (Family: none) | |

Form PCT/ISA/210 (patent family annex) (January 2015)

**EP 4 238 547 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013124250 A